(19) Europäisches Patentamt
European Patent Office
Office européen des brevets

(11) **EP 4 008 256 A1**

(12) **EUROPEAN PATENT APPLICATION**

(43) Date of publication:
**08.06.2022 Bulletin 2022/23**

(21) Application number: **20211336.1**

(22) Date of filing: **02.12.2020**

(51) International Patent Classification (IPC):
***A61B 6/00*** (2006.01)     ***G21K 1/02*** (2006.01)
***G21K 1/04*** (2006.01)

(52) Cooperative Patent Classification (CPC):
**A61B 6/4291; G21K 1/025; G21K 1/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **alephFS - Systems for Imaging 3461217 Haifa (IL)**

(72) Inventor: **LEVINSON, Reuven 3461217 Haifa (IL)**

(74) Representative: **Boult Wade Tennant LLP Salisbury Square House 8 Salisbury Square London EC4Y 8AP (GB)**

(54) **ANTI-SCATTER GRID FOR X-RAY IMAGING**

(57) Apparatus and methods for use with an X-ray system are described. An X-ray anti-scatter grid (22) includes at least a first layer (11a) of elongate radiopaque septa (10) arranged such that longitudinal axes of each the septa (10) belonging to the first layer are disposed along a first direction, in parallel to each other, spaces between the septa (10) of the layer being filled with air. Two or more slotted plates (16) are coupled to a rigid frame (9), each of the slotted plates defining a plurality of slots (17). A pulling mechanism (20) applies tension to the plurality of septa (10) that are arranged in parallel to each other, during use of the X-ray anti-scatter grid, such that, along lengths of the septa (10), longitudinal axes of the septa (10) are maintained in a parallel configuration with respect to each other. Other applications are also described.

FIG.9

EP 4 008 256 A1

**Description**

**FIELD OF EMBODIMENTS OF THE INVENTION**

Applications of the present invention include an anti-scatter grid for use in medical projection X-ray imaging. More particularly, applications of the present invention relate to an air-filled anti-scatter grid for reducing the X-ray scatter radiation incident on the X-ray detector.

**BACKGROUND**

In transmission X-ray imaging, X-rays produced by an X-ray tube, penetrate an object and are then absorbed in an X-ray detector, leading to the creation of the X-ray image. X-rays that penetrate the object without interacting with the object (these rays travel on a straight-line path from the tube to the detector) are called primary X-rays. X-rays that penetrate the object after interacting with the object (and have been diverted from the original straight line path) are called scattered X-rays. Primary X-rays absorbed by the X-ray detector produce the primary image. Scattered X-rays absorbed by the X-ray detector produce the scatter image. The primary and scatter X-ray images are superimposed on each other and together produce the X-ray image. The primary image contains all the information of the X-ray image. The scatter image does not contain any useful information and reduces the signal-to-ratio (SNR) of the X-ray image. Therefore, for maximum SNR in the X-ray image, it is desired to reduce the number of scattered X-rays absorbed in the X-ray detector to a minimum while simultaneously preserving the number of primary X-rays absorbed in the X-ray detector.

Grids reduce the number of scattered X-rays absorbed in the X-ray detector. Grids also reduce the number of primary X-rays absorbed in the X-ray detector. Two performance metrics of grids are scattered X-ray transmission (Ts) and primary X-ray transmission (Tp). The measurement of these 2 metrics is described in the IEC document 60627: Diagnostic X-ray imaging equipment -Characteristics of general purpose and mammographic anti-scatter grids. An ideal grid has: Tp =1 and Ts = 0. Typically, commercial grids have: Tp in the range of 0.6 to 0.8, and Ts in the range of 0.07 to 0.2. As a result of the transmitted scattered radiation and the reduction in the primary radiation, the SNR of the X-ray image is reduced by a factor of (versus an ideal grid):

$$ SQRT\ [Tp/(1 + SPR(Ts/Tp))], $$

where SPR is the ratio of the number of scattered X-rays to the number of primary X-rays incident on the X-ray detector without any grid.

In projection X-ray imaging of the abdomen of adult humans the SPR is approximately 10. For abdominal imaging, with grid parameters: Tp=0.7 and Ts =0.1, the reduction in the image SNR is a factor of 0.55.

Medical projection X-ray imaging is performed with an X-ray tube having a 'point source' with produces a divergent x-ray beam. Due to the divergent beam geometry, grids have a 'focused geometry' and are called focused grids. Grid septa are configured so that the orientation of the height dimension of the grid septa is parallel to the direction of the primary X-rays. This configuration maximizes the transmission of the primary X-rays.

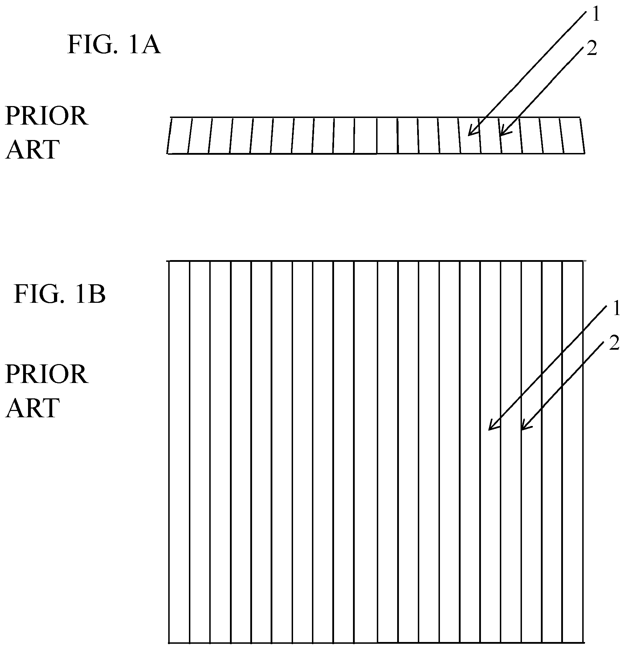

Reference is now made to Figs. 1A and 1B, which are schematic illustrations of, respectively, a side view and a top view of a prior art X-ray grid. As shown in Figs. 1A and 1B, prior art grids typically have septa 2 and interspace material 1. The septa are thin foils of high atomic number material (such as lead). The interspace material is low atomic number material (such as, aluminum) and fills the space between the septa. Typically, such prior art grids require a solid material to be used as the interspace material, because the septa are thin and require the support of the solid interspace material in order to maintain their shapes.

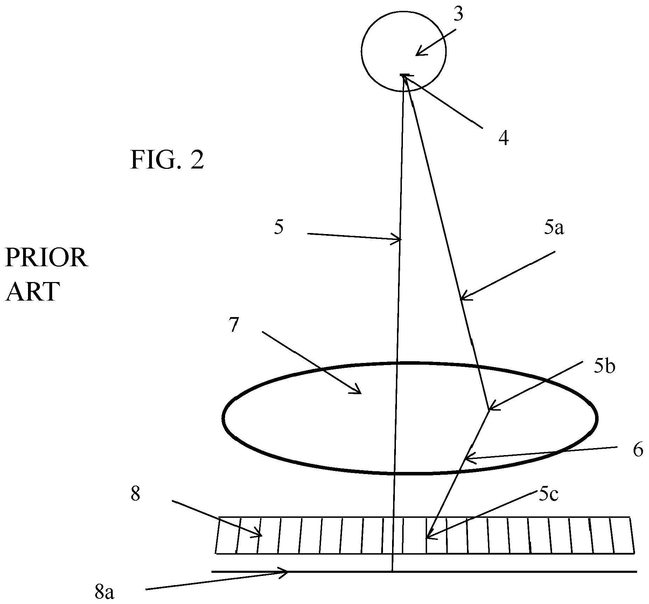

Reference is also made to Fig. 2, which is a schematic illustration of a side view of a projection imaging configuration, in accordance with prior art techniques. An X-ray tube 3 contains an X-ray focal spot 4, which emits all the primary X-rays used in projection imaging.

A first primary X-ray 5 is emitted from the focal spot, transverses the patient 7 and grid 8 and is absorbed in the X-ray detector 8a. A second primary X-ray 5a interacts with the patient 7 at point 5b. A scattered X-ray 6 is emitted from the interaction point and is absorbed in the grid septa at point 5c.

The function of the septa is to absorb the scattered X-rays. The grid is placed in the X-ray beam so that the septa are parallel to the direction of the primary X-rays. In order to preferentially absorb scattered X-rays and transmit primary X-rays the septa are typically thin foils, placed parallel to the direction of the primary X-rays (in order to minimize primary X-ray absorption), and made from high density, high atomic number material (in order to maximize scattered X-ray absorption).

**SUMMARY OF EMBODIMENTS**

In accordance with some applications of the present invention, an X-ray anti-scatter grid for use with an X-ray system includes at least a first layer of elongate radiopaque septa arranged such that longitudinal axes of each the septa belonging to the first layer are disposed along a first direction, in parallel to each other, spaces between the septa of the layer being filled with air. The elongate septa are coupled to a rigid frame, such that the rigid frame supports the elongate septa. Typically, the rigid frame and the elongate septa are not formed as an integral unit, for example, via a three-dimensional printing process. For some applications, two or more slotted plates are coupled to the rigid frame, with each of the slotted plates defining a plurality of slots. Each of the septa typically passes through a respective pair of slots defined by a pair of the slotted plates disposed on opposite sides of the frame from each other, such that an orientation of each of the septa with respect to the frame is determined by an orientation of the corresponding pair of slots with respect to the frame.

For some applications, the X-ray anti-scatter grid includes a second layer of elongate radiopaque septa, arranged such that longitudinal axes of each the septa belonging to the second layer are disposed along a second direction, in parallel to each other. The second direction is typically perpendicular to the first direction, such that, when viewed along a third direction that is perpendicular to the first and second directions, the first and second layers of radiopaque septa define a grid.

For some applications, a controller receives an input indicating a focal length of the X-ray system, and, in response thereto, the controller adjusts orientations of the septa within the first layer. For some applications, the controller adjusts orientations of the septa within the first layer by adjusting orientations of the slotted plates with respect to the frame, e.g., using a stepper motor.

There is therefore provided, in accordance with some applications of the present invention, apparatus for use with an X-ray system including:
an X-ray anti-scatter grid including:

> at least a first layer of elongate radiopaque septa arranged such that longitudinal axes of each the septa belonging to the first layer are disposed along a first direction, in parallel to each other, spaces between the septa of the layer being filled with air;
> a rigid frame configured to support the elongate septa; and
> two or more slotted plates coupled to the rigid frame, each of the slotted plates defining a plurality of slots, each of the septa passing through a respective pair of slots defined by a pair of the slotted plates disposed on opposite sides of the frame from each other, such that an orientation of each of the septa with respect to the frame is determined by an orientation of the corresponding pair of slots with respect to the frame.

In some applications, the rigid frame and the elongate septa are not formed as an integral unit. In some applications, the rigid frame and the elongate septa are not formed via a three-dimensional printing process.

In some applications, the X-ray anti-scatter grid includes a single layer of elongate radiopaque septa. In some applications, the X-ray anti-scatter grid further includes a second layer of elongate radiopaque septa, arranged such that longitudinal axes of each the septa belonging to the second layer are disposed along a second direction, in parallel to each other, the second direction being perpendicular to the first direction, such that, when viewed along a third direction that is perpendicular to the first and second directions, the first and second layers of radiopaque septa define a grid.

In some applications, a thickness of each of the septa is between 0.04 mm and 0.1 mm. In some applications, a height of each of the septa is between 10 mm and 20 mm. In some applications, within the layer of septa, a distance between each of the septa and adjacent septa is between 0.5 mm and 1.5 mm.

In some applications, the apparatus further includes a controller configured to:

> receive an input indicating a focal length of the X-ray system, and
> in response thereto, to adjust orientations of the septa within the first layer.

In some applications, the controller is configured to adjust orientations of the septa within the first layer such that the septa are parallel to a primary X-ray beam generated by the X-ray system.

In some applications, the X-ray system includes an X-ray system processor, and wherein the controller is configured to automatically receive the input indicating the focal length of the X-ray system from the X-ray system processor. In some applications, the controller is configured to manually receive the input indicating the focal length of the X-ray system. In some applications, the controller is configured to adjust orientations of the septa within the first layer by adjusting orientations of the slotted plates with respect to the frame. In some applications, the apparatus further includes one or more stepper motors, and the controller is configured to adjust orientations of the septa within the first layer by adjusting orientations of the slotted plates with respect to the frame using the stepper motor.

In some applications, each of the septa includes a foil core that is coated with a metal that has an atomic number that is greater than an atomic number of the foil core. In some applications, the foil core includes a copper foil core. In some applications, the metal coating includes tin.

There is further provided, in accordance with some applications of the present invention, a method including:

> driving an X-ray system to direct X-rays from an X-ray source to an X-ray detector; and

> directing the X-rays via an X-ray anti-scatter grid that includes:

>> at least a first layer of elongate radiopaque septa arranged such that longitudinal axes of each the septa belonging to the first layer are disposed along a first direction, in parallel to each other, spaces between the septa of the layer being filled with air;
>> a rigid frame configured to support the elongate septa; and

two or more slotted plates coupled to the rigid frame, each of the slotted plates defining a plurality of slots, each of the septa passing through a respective pair of slots defined by a pair of the slotted plates disposed on opposite sides of the frame from each other, such that an orientation of each of the septa with respect to the frame is determined by an orientation of the corresponding pair of slots with respect to the frame.

There is further provided, in accordance with some applications of the present invention, apparatus for use with an X-ray system including:
an X-ray anti-scatter grid comprising:

at least a first layer of elongate radiopaque septa that comprises a plurality of elongate radiopaque septa arranged such that longitudinal axes of each the septa belonging to the first layer are disposed along a first direction, in parallel to each other, spaces between the septa of the layer being filled with air; and
a controller configured to:

receive an input indicating a focal length of the X-ray system, and
in response thereto, to adjust orientations of the septa within the layer.

In some applications, the controller is configured to adjust orientations of the septa within the first layer such that the septa are parallel to a primary X-ray beam generated by the X-ray system.
In some applications, wherein the X-ray system includes an X-ray system processor, and wherein the controller is configured to automatically receive the input indicating the focal length of the X-ray system from the X-ray system processor.
In some applications, wherein the controller is configured to manually receive the input indicating the focal length of the X-ray system.
In some applications, wherein the X-ray anti-scatter grid includes a single layer of elongate radiopaque septa.
In some applications, the X-ray anti-scatter grid further includes a second layer of elongate radiopaque septa, arranged such that longitudinal axes of each the septa belonging to the second layer are disposed along a second direction, in parallel to each other, the second direction being perpendicular to the first direction, such that, when viewed along a third direction that is perpendicular to the first and second directions, the first and second layers of radiopaque septa define a grid.
In some applications, a thickness of each of the septa is between 0.04 mm and 0.1 mm. In some applications, a height of each of the septa is between 10 mm and 20 mm. In some applications, within the layer of septa, a distance between each of the septa and adjacent septa is between 0.5 mm and 1.5 mm.
In some applications, the X-ray anti-scatter grid further includes:

a rigid frame configured to support the elongate septa; and
two or more slotted plates coupled to the rigid frame, each of the slotted plates defining a plurality of slots, each of the septa passing through a respective pair of slots defined by the plates, such that an orientation of each of the septa with respect to the frame is determined by an orientation of the corresponding pair of slots with respect to the frame.

In some applications, the controller is configured to adjust orientations of the septa within the first layer by adjusting orientations of the slotted plates with respect to the frame. In some applications, the apparatus further includes one or more stepper motors, and the controller is configured to adjust orientations of the septa within the first layer by adjusting orientations of the slotted plates with respect to the frame using the stepper motor.
In some applications, each of the septa includes a foil core that is coated with a metal that has an atomic number that is greater than an atomic number of the foil core. In some applications, the foil core includes a copper foil core. In some applications, the metal coating includes tin.
There is further provided, in accordance with some applications of the present invention, a method for use with an X-ray system that is configured to direct X-rays from an X-ray source to an X-ray detector, the method including:

directing the X-rays via an X-ray anti-scatter grid that include at least a first layer of elongate radiopaque septa that comprises a plurality of elongate radiopaque septa arranged such that longitudinal axes of each the septa belonging to the first layer are disposed along a first direction, in parallel to each other, spaces between the septa of the layer being filled with air; and
in response to an indication of a focal length of the X-ray system, causing orientations of the septa within the layer to be adjusted.

There is further provided, in accordance with some applications of the present invention, apparatus for use with an X-ray system including:
an X-ray anti-scatter grid comprising:

at least a first layer of elongate radiopaque septa arranged such that longitudinal axes of each the septa belonging to the first layer are disposed along a first direction, in parallel to each other, spaces between the septa of the layer being filled with air;
a rigid frame to which the elongate septa are coupled, such that the rigid frame supports the elongate septa, the rigid frame and the elongate septa not being formed as an integral unit.

There is further provided, in accordance with some applications of the present invention, apparatus for use with an X-ray system including:
an X-ray anti-scatter grid comprising:

at least a first layer of elongate radiopaque septa arranged such that longitudinal axes of each the septa belonging to the first layer are disposed along a first direction, in parallel to each other, spaces between the septa of the layer being filled with air;
a rigid frame to which the elongate septa are coupled, such that the rigid frame supports the elongate septa, the rigid frame and the elongate septa not being formed via a three-dimensional printing process.

There is further provided, in accordance with some applications of the present invention, apparatus for use with an X-ray system including:
an X-ray anti-scatter grid including:

at least a first layer of elongate radiopaque septa arranged such that longitudinal axes of each the septa belonging to the first layer are disposed along a first direction, in parallel to each other, spaces between the septa of the layer being filled with air;
a rigid frame;
two or more slotted plates coupled to the rigid frame, each of the slotted plates defining a plurality of slots, each of the septa passing through a respective pairs of slots defined by a pair of the slotted plates disposed on opposite sides of the frame from each other, such that an orientation of each of the septa with respect to the frame is determined by an orientation of the corresponding pair of slots with respect to the frame; and
a pulling mechanism configured to apply tension to the plurality of septa that are arranged in parallel to each other, during use of the X-ray anti-scatter grid, such that, along lengths of the septa, longitudinal axes of the septa are maintained in a parallel configuration with respect to each other.

In some applications, the rigid frame and the elongate septa are not formed as an integral unit.
In some applications, the X-ray anti-scatter grid further includes a second layer of elongate radiopaque septa, arranged such that longitudinal axes of each the septa belonging to the second layer are disposed along a second direction, in parallel to each other, the second direction being perpendicular to the first direction, such that, when viewed along a third direction that is perpendicular to the first and second directions, the first and second layers of radiopaque septa define a grid. In some applications, the X-ray anti-scatter grid includes a single layer of elongate radiopaque septa.

In some applications, a thickness of each of the septa is between 0.04 mm and 0.1 mm. In some applications, a height of each of the septa is between 10 mm and 20 mm. In some applications, within the layer of septa, a distance between each of the septa and adjacent septa is between 0.5 mm and 1.5 mm.
In some applications, two or more slotted plates are disposed at each end of the septa, and the slots defined by the two or more slotted plates disposed at each end of the septa are misaligned with respect to each other, such as to trap the ends of the septa within the slots defined by the slotted plates. In some applications, the ends of the septa defines holes therethrough, and one or more rods are disposed within the holes defined by the septa such as to longitudinally align the septa with respect to each other.
In some applications, the apparatus further includes a controller configured to:

receive an input indicating a focal length of the X-ray system, and
in response thereto, to adjust orientations of the septa within the first layer.

In some applications, the controller is configured to adjust orientations of the septa within the first layer such that the septa are parallel to a primary X-ray beam generated by the X-ray system. In some applications, the controller is configured to adjust orientations of the septa within the first layer by adjusting orientations of the slotted plates with respect to the frame.
In some applications, the pulling mechanism includes:

at least one septa-supporting element that defines a cavity that is configured to receive the ends of the septa and one of the pair of slotted plates; and
a plurality of tension-applying elements disposed between the septa-supporting element and a portion of the frame.

In some applications, the plurality of tension-applying elements includes a plurality of screws, the screws being tightened to a sufficient extent that the septa are maintained in taut configurations, such that, along the lengths of the septa, the longitudinal axes of the septa are maintained in a parallel configuration with respect to each other. In some applications, the apparatus further includes adhesive disposed within the cavity defined by the septa-supporting element and configured to adhere the ends of the septa and the slotted plates to an interior of the septa-supporting element.
There is further provided, in accordance with some applications of the present invention, apparatus for use with an X-ray system including:
an X-ray anti-scatter grid including:

at least a first layer of elongate radiopaque septa

arranged such that longitudinal axes of each the septa belonging to the first layer are disposed along a first direction, in parallel to each other, spaces between the septa of the layer being filled with air;
a rigid frame;
two or more slotted plates coupled to the rigid frame, each of the slotted plates defining a plurality of slots, each of the septa passing through a respective pairs of slots defined by a pair of the slotted plates disposed on opposite sides of the frame from each other, such that an orientation of each of the septa with respect to the frame is determined by an orientation of the corresponding pair of slots with respect to the frame;
at least one septa-supporting element that defines a cavity that is configured to receive the ends of the septa and one of the pair of slotted plates; and
a plurality of tension-applying elements disposed between the septa-supporting element and a portion of the frame.

In some applications, the plurality of tension-applying elements includes a plurality of screws, the screws being tightened to a sufficient extent that the septa are maintained in taut configurations, such that, along the lengths of the septa, the longitudinal axes of the septa are maintained in a parallel configuration with respect to each other. In some applications, the apparatus further includes adhesive disposed within the cavity defined by the septa-supporting element and configured to adhere the ends of the septa and the slotted plates to an interior of the septa-supporting element.

In some applications, the rigid frame and the elongate septa are not formed as an integral unit.

In some applications, the X-ray anti-scatter grid further includes a second layer of elongate radiopaque septa, arranged such that longitudinal axes of each the septa belonging to the second layer are disposed along a second direction, in parallel to each other, the second direction being perpendicular to the first direction, such that, when viewed along a third direction that is perpendicular to the first and second directions, the first and second layers of radiopaque septa define a grid. In some applications, the X-ray anti-scatter grid includes a single layer of elongate radiopaque septa.

In some applications, a thickness of each of the septa is between 0.04 mm and 0.1 mm. In some applications, a height of each of the septa is between 10 mm and 20 mm. In some applications, within the layer of septa, a distance between each of the septa and adjacent septa is between 0.5 mm and 1.5 mm.

In some applications, two or more slotted plates are disposed at each end of the septa, and the slots defined by the two or more slotted plates disposed at each end of the septa are misaligned with respect to each other, such as to trap the ends of the septa within the slots defined by the slotted plates. In some applications, the ends of the septa defines holes therethrough, and one or more rods are disposed within the holes defined by the septa such as to longitudinally align the septa with respect to each other.

In some applications, the apparatus further includes a controller configured to:

receive an input indicating a focal length of the X-ray system, and
in response thereto, to adjust orientations of the septa within the first layer.

In some applications, the controller is configured to adjust orientations of the septa within the first layer such that the septa are parallel to a primary X-ray beam generated by the X-ray system. In some applications, the controller is configured to adjust orientations of the septa within the first layer by adjusting orientations of the slotted plates with respect to the frame.

The present invention will be more fully understood from the following detailed description of embodiments thereof, taken together with the drawings, in which:

**BRIEF DESCRIPTION OF THE DRAWINGS**

Figs. 1A and 1B are schematic illustrations of a side view and top view of a focused grid with interspace material and septa, in accordance with prior art techniques;

Fig. 2 is a schematic illustration of a side view of a projection imaging configuration, in accordance with prior art techniques;

Figs. 3A and 3B are schematic illustrations of, respectively, a side view and a top view of a grid, in accordance with some applications of the present invention;

Figs. 4A and 4B are schematic illustrations of, respectively, a side view and top view of the septa of a grid, in accordance with some applications of the present invention;

Figs. 5A and 5B are schematic illustrations of, respectively, a side view and top view of a slotted plate, in accordance with some applications of the present invention;

Fig. 6 is a schematic illustration of a top view of a grid including septa and a frame, in accordance with some applications of the present invention;

Fig. 7 is a schematic illustration of a grid of septa placed in an X-ray beam and oriented such that the septa are parallel to the direction of the primary X-ray beams, in accordance with some applications of the present invention;

Fig. 8 is a schematic illustration of a slotted plate mounted on a rotation axis, in accordance with some applications of the present invention;

Fig. 9 is a schematic illustration of a grid that is configured to operate at multiple focal lengths, in accordance with some applications of the present invention;

Figs. 10A and 10B are schematic illustrations of respective views of a grid during an initial stage of the construction of the grid, in accordance with some applications of the present invention;

Figs. 11A and 11B are schematic illustrations of respective views of a grid during a subsequent stage of the construction of the grid, in accordance with some applications of the present invention;

Figs. 12A and 12B are schematic illustrations of respective views of a grid once the grid has been fully constructed, in accordance with some applications of the present invention;

Figs. 13A, 13B, and 13C are schematic illustrations of a septa-supporting element of a pulling mechanism of an x-ray grid, in accordance with some applications of the present invention;

Figs. 14A, 14B, and 14C are schematic illustrations of the septa-supporting element of Figs, 13A, 13B, and 13C with additional components of the X-ray anti-scatter grid, in accordance with some applications of the present invention; and

Fig. 15 is a schematic illustration of a fully-assembled X-ray anti-scatter grid that includes a frame and a pulling mechanism, in accordance with some applications of the present invention.

## DETAILED DESCRIPTION OF EMBODIMENTS

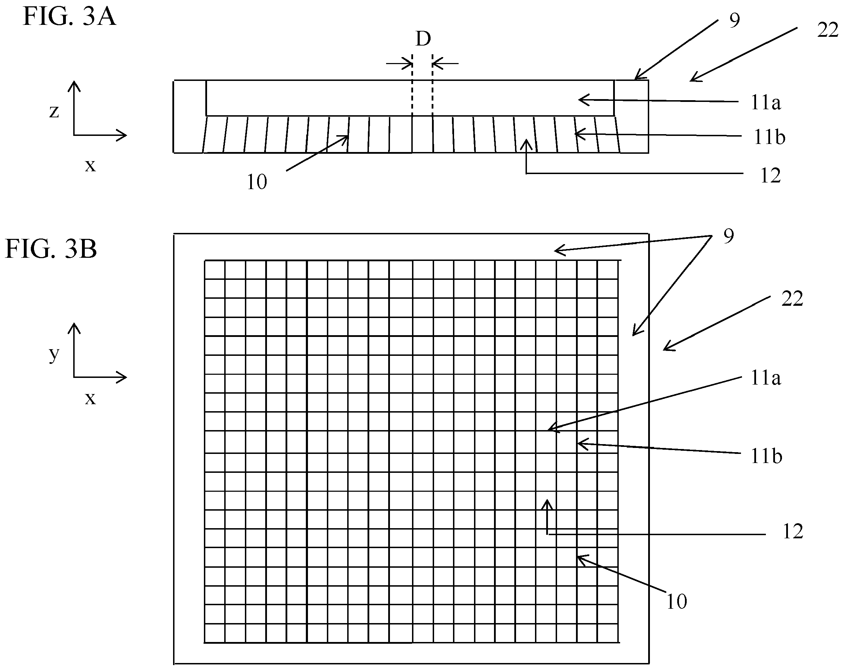

Reference is now made to Figs. 3A and 3B, which are schematic illustrations of an X-ray anti-scatter grid 22, in accordance with some applications of the present invention. Fig. 3A shows a side view of the grid (looking along the y-direction), and Fig. 3B shows a top view of the grid (looking along the z-direction). For some applications, grid 22 includes first and second layers of elongate radiopaque septa 10, e.g., top layer 11a (closer to the X-ray focal spot) and bottom layer 11b. The first layer of elongate radiopaque septa includes a plurality of elongate radiopaque septa 10 arranged such that longitudinal axes of each the septa belonging to the first layer are disposed along a first direction (e.g., the x-direction), in parallel to each other. The second layer of elongate radiopaque septa includes a plurality of elongate radiopaque septa 10 arranged such that longitudinal axes of each the septa belonging to the second layer are disposed along a second direction, in parallel to each other, the second direction being generally perpendicular to the first direction (e.g., the y-direction). The first layer of radiopaque septa are disposed above the second layer of radiopaque septa, such that, when viewed along a third direction that is perpendicular to the first and second directions (e.g., the z-direction), the first and second layers of radiopaque septa define a grid. The septa are maintained in their respective positions within grid 22 by a rigid frame 9 that is disposed around the outside of the grid and supports the ends of the septa. It is noted that the frame and the septa are typically separate components that are coupled to each other, and are not made of a single integral structure (e.g., via a 3D printing process). The interspace material 12 (i.e., the spaces between the septa of each of the layers) is typically air. For some applications, within each of the layers of septa, a distance D (shown in Fig. 3A) between each of the septa and adjacent septa (also known as the "pitch" of the grid) is between 0.5 mm and 1.5 mm.

Each layer of septa is a linear, focused air-filled grid. The two layers are disposed such that, together, they form a crisscross grid. As shown in Fig. 3B, the septa of the two layers (top layer 11a and bottom layer 11b) are perpendicular to each other.

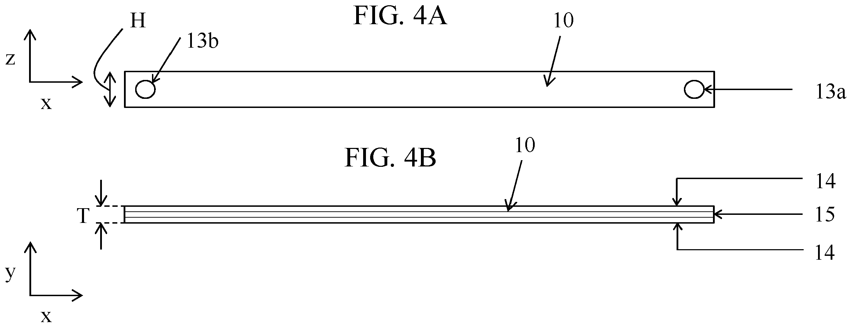

Reference is now made to Figs. 4A-B, which show the structure of an individual septum 10, in accordance with some applications of the present invention. Fig. 4A shows a side view of a septum (looking along the y-direction), and Fig. 4B shows a top view of the septum (looking along the z-direction). For some applications, the septa are made of a core 15 and a coating 14 that coats the core. Typically, the core provides rigidity to the septa and the coating provides X-ray absorption. For some applications, the core is a foil core and the coating is a metal that has an atomic number that is greater than the atomic number of the foil core. For example, the core may be copper and/or steel, and the coating may be tin. For some applications, the septa are made of tungsten and/or phosphor bronze. For example, all of the septa may be made of either tungsten or phosphor bronze. Alternatively, septa made of tungsten and septa made of phosphor bronze may be alternated with one another (e.g., such that odd-numbered septa are made of tungsten and even-numbered septa are made of phosphor bronze, or vice versa), or used with each other in a different combination. Typically, for applications in which the septa are made of tungsten and/or phosphor bronze, the septa are not constructed using separate materials for cores and coatings of the septa. Rather, each of the septa is typically made of either one or the other of these materials.

Typically, the septa define holes 13a and 13b in the ends of the septa for connection to rods, as described hereinbelow. For some applications, the rods are circular, and holes 13a and 13b are circular (as shown) to conform with the circular rods.

For some applications, a thickness T of each of the septa 10 is greater than 0.04 mm and/or less than 0.1 mm, e.g., between 0.04 mm and 0.1 mm. For some applications, a height H of each of the septa is greater than 10 mm and/or less than 20 mm, e.g., between 10 mm and 20 mm.

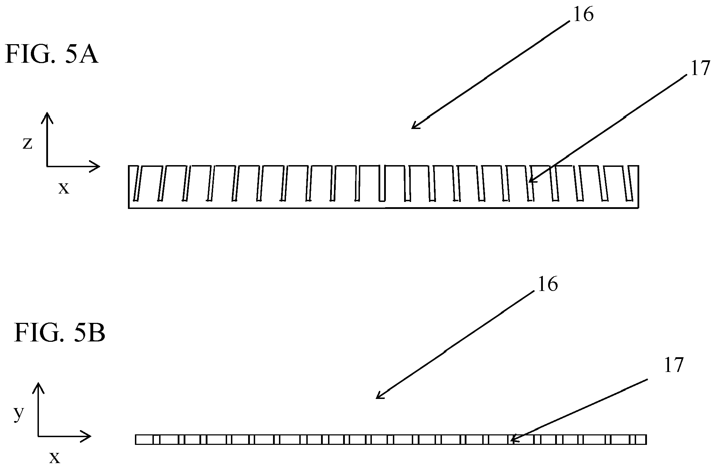

Reference is now made to Figs. 5A and 5B, which are schematic illustrations of respective views of a slotted plate 16, in accordance with some applications of the present invention. Fig. 5A shows a side view of the plate (looking along the y-direction), and Fig. 5B shows a top view of the plate (looking along the z-direction). For some applications, the septa are held in place, at each end, by the slots 17 defined by the slotted plates. The distance between the septa and the orientation of the septa in the grid is set by slots in the slotted plates. The width of the slots is typically slightly larger than the thickness of the septa. The slotted plates typically are coupled to rigid frame 9. Typically, each of the septa passes through a respective pair of slots, such that an orientation of each of the septa with respect to the frame is determined by an orientation of the corresponding pair of slots with respect to the frame .

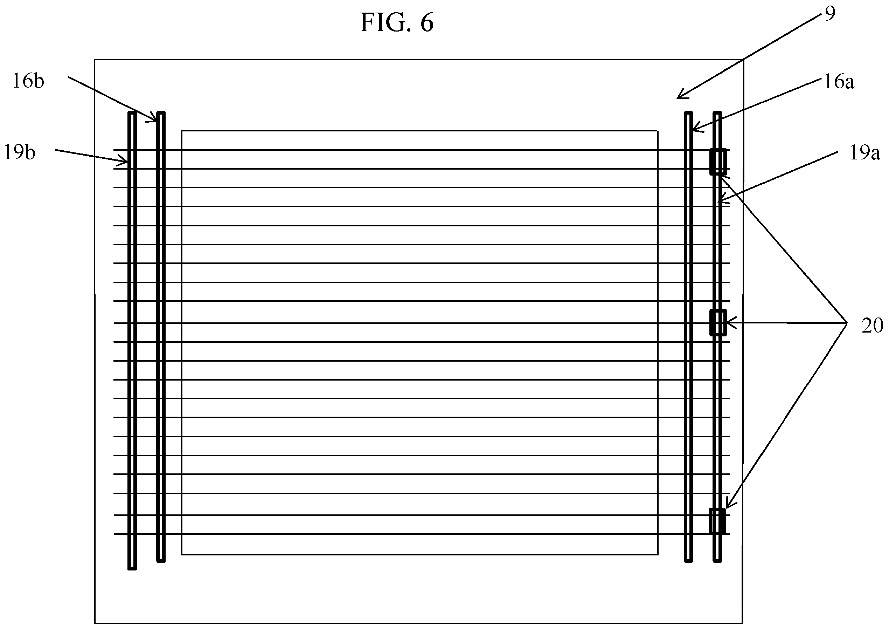

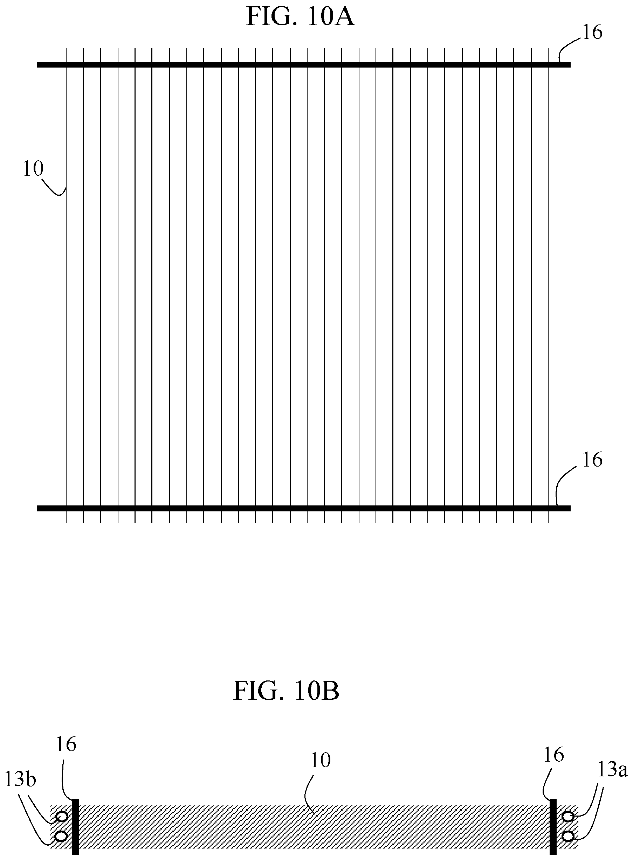

Reference is now made to Fig. 6, which is a schematic illustration of septa 10 from one layer being maintained in position by rigid frame 9, in accordance with some applications of the present invention. As described hereinabove, for some applications, the septa are held in place, at each end, by the slots defined by slotted plates 16a, 16b that are disposed on or within rigid frame 9. For some applications, the ends of the septa are attached to rods 19a, 19b, for example, via holes 13a, 13b in the ends of the septa, as shown in Fig. 4A. For some applications, the rods are circular. For some applications, the rods are disposed upon frame 9. One of the rods (e.g., rod 19a) is moved away from the second rod (e.g., rod 19b) so that the septa are tightly stretched between the rods. For some applications, a pulling mechanism 20 is disposed upon frame 9 and the rod is pulled using the pulling mechanism. For some applications, the slotted plates, the rods, and the frame are arranged with respect to each other in a configuration as described hereinbelow with reference to Figs. 10A-15.

It is noted that grid 22 as shown in Figs. 3A-6 is configured such that (a) the septa are supported in their positions by elements that are disposed on or within rigid frame 9 (e.g., slotted plates 16a, 16b as described hereinabove), and (b) between the support elements that are disposed upon the rigid frame the septa have a sufficient amount of rigidity to support themselves (e.g., by virtue of the fact that the septa are constructed from a core and a coating, as described hereinabove). Typically, the grid is configured, such that when the grid is used in X-ray imaging, the frame itself is not within the field of view of the X-ray detector. As such, all of the support elements that support the septa in place are disposed outside of the field of view of the X-ray detector, and do not absorb any X-rays.

As described hereinabove, typically within each of the layers of septa, spaces between the septa are filled with air. For some applications, this provides an advantage over conventional grids (in which a low atomic number solid material (such as aluminum) is typically used as the interspace material, as described hereinabove), because air does not absorb any X-rays whereas the interspace material that is used in conventional grids typically does absorb some X-rays. Moreover, since the interspace material on conventional grids absorbs X-rays, this typically places an upper limit on the heights of the septa that may be used in the grid. By contrast, since the interspace material in grid 22 is air, grid 22 is not limited in this manner. Therefore, typically, the height H of each of the septa is greater than 10 mm and/or less than 20 mm, e.g., between 10 mm and 20 mm. In turn, for some applications, by virtue of septa having the aforementioned heights, the distance D (shown in Fig. 3A) between each of the septa and adjacent septa (also known as the "pitch" of the grid) may be between 0.5 mm and 1.5 mm, and the grid may still have a grid ratio of more than 10, or more than 15 (grid ratio being defined as the ratio of the heights of the septa to the pitch of the grid, i.e., H:D). Since conventional grids typically have an upper limit on the height of the septa, e.g., for the reasons provided hereinabove, it is typically the case that there is a corresponding upper limit on the pitch of the grid, in order to provide such a grid ratio.

Although the grid shown in Figs. 3A-6 shows two layers of septa that are disposed perpendicularly with respect to each other such as to form a grid, for some applications, grid 22 is configured as described herein, but includes only a single layer of septa, the longitudinal axes of which are parallel to each other. Typically, such a grid would include either layer 11a or 11b of septa. Typically, for such applications, the height H of each of the septa is still greater than 10 mm and/or less than 20 mm, e.g., between 10 mm and 20 mm. In all other aspects, such a grid would be generally similar to that described hereinabove.

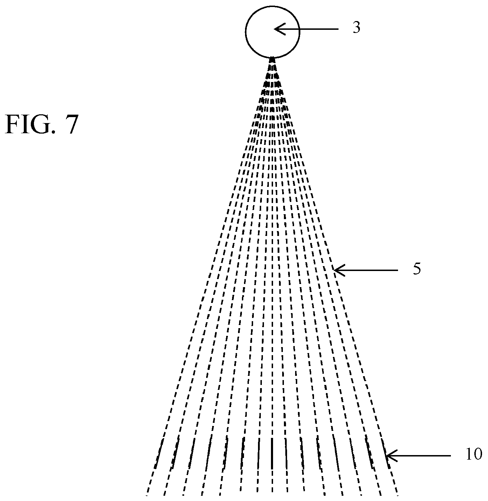

Reference is now made to Fig. 7, which is a schematic illustration of a grid 22 of septa 10 placed in an X-ray beam and oriented such that the septa are parallel to the direction of the primary X-ray beams 5, in accordance with some applications of the present invention. As shown, typically, the X-ray beam spreads in a fan, such that the primary X-ray beams are disposed at respective angles to the axis of X-ray source 3. Typically, conventional grids are suitable for use with a specific focal spot-detector length (i.e., focal length). If a focused grid is used in an imaging examination does not correspond to the focal length of the grid, primary beam cut-off occurs where the transmission of the primary beam is reduced and thus the performance of the grid is degraded. However, diagnostic X-ray systems typically perform X-ray examinations at different focal lengths. When a fixed focal length grid is used with X-ray examinations of different focal lengths, the grid performance is degraded. Conversely, if different X-ray examinations at different focal lengths utilize multiple grids (with different focal lengths), this requires an operator to manually change the grids for the different X-ray examinations. Therefore, in accordance with some applications of the present invention, grid 22 is configured such as to be adjustable such as to operate at multiple focal lengths.

For some applications, the orientation of the grid septa are adjustable such that the grid is configured to operate at multiple focal lengths. Typically, such applications are applicable to a grid that includes two layers of septa as described hereinabove, or to a grid that includes only a single layer of septa.

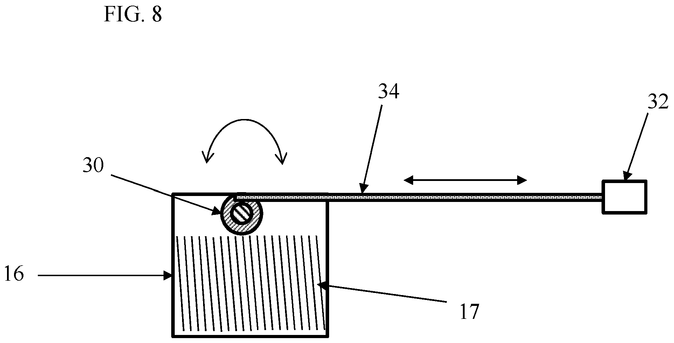

Reference is now made to Fig. 8, which is a schematic illustration of slotted plate 16 (e.g., slotted plate 16a or slotted plate 16b, described hereinabove) mounted on a rotation axis 30, in accordance with some applications of the present invention. For some applications, an actuator 32 is used to mechanically adjust the orientation of the septa. For example, the actuator may be a stepper motor that is coupled to the septa via a gear drive 34. Typically, the orientation of the septa is determined by the orientations of slots 17 in slotted-plates 16 (described hereinabove with reference to Figs. 5A and 5B), and by the orientations of the slotted plates. For some applications, each of the septa is held within slots of a pair of slotted plates, the slotted plates being disposed at respective ends of the septum. Typically, the angle of the septum with respect to the slotted plates (and therefore the angles of the septa with respect to the frame) is fixed by the orientations of the corresponding pair of slots 17 in the pair of slotted-plates 16. For some applications, the orientations of the slotted plates is controlled by actuator 32. For some applications, the range of the rotation motion of the slotted plates is up to +/- 5 degrees from a central position of the slotted plates. The rotation of the slotted plates typically results in a rotation of the orientation of the septa which adjusts the focal length of the grid.

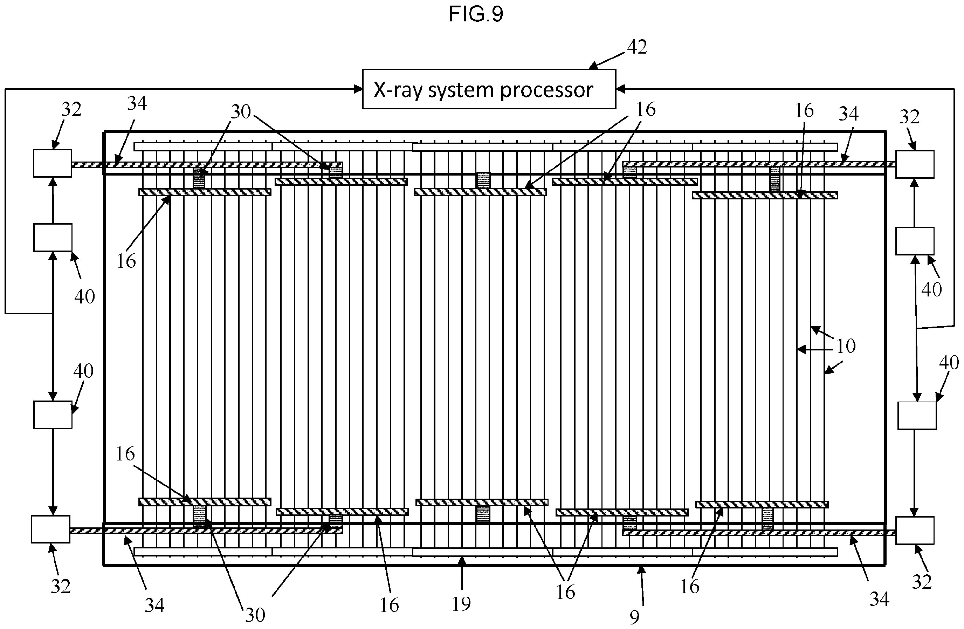

Reference is now made to Fig. 9, which is a schematic illustration of grid 22, grid 22 being configured to operate at multiple focal lengths, in accordance with some applications of the present invention. For some applications, grid 22 includes multiple groups of septa 10 aligned adjacent to each other to cover the entire field of view of the x-ray beam. For some applications, an electronic controller 40 (e.g., circuitry and a processor) is configured to receive focal length inputs from an X-ray system processor 42. Typically, in response thereto, the electronic controller drives actuators 32 that are associated with respective of slotted plates 16 to adjust the orientations of the slotted plates, such that each group of septa is oriented in an orientation that is suitable for the received focal length inputs. This method of automatically changing the orientation of the grid septa as described herein typically enables the use of the grid in X-ray examinations with different focal lengths without any need for operator intervention.

Typically, the slotted-plates 16 and the rods 19 are physically coupled to rigid frame 9, such that the frame provides mechanical stability for the slotted plates, the rods, and the septa. One or more motors (e.g., four motors, as shown in the X-ray system of Fig. 9) are typically used to drive the rotation of the slotted-plates. For some applications, each group of septa, held in place by a pair of slotted plates, is rotated to a respective different angle (depending on the position of the group of septa in the grid), such as to produce the required orientation for each group of septa as a function of the required focal length. Typically, the one or more motors are connected to the X-ray system processor (e.g., via controllers 40) and receive the focal length input information from the X-ray system for adjusting the orientation of the septa, to dynamically change the configuration of the variable focus grid in response to the input information. Typically, the motors are configured to drive the slotted plates to adjust their orientations by driving rotation axes 30 to rotate via gear drives 34. For some applications, a single motor is configured to control more than one rotation axis, as shown in Fig. 9. For some applications, controllers 40 are configured to send an input to the X-ray system processor, e.g., to transmit a ready status to the X-ray system processor.

For some applications, generally similar techniques to those described with reference to Fig. 9 are performed, but a human operator (a) manually inputs the focal length of the X-ray system to controllers 40, and/or (b) manually instructs the X-ray system to acquire X-rays, once the grid has been correctly configured by controllers 40.

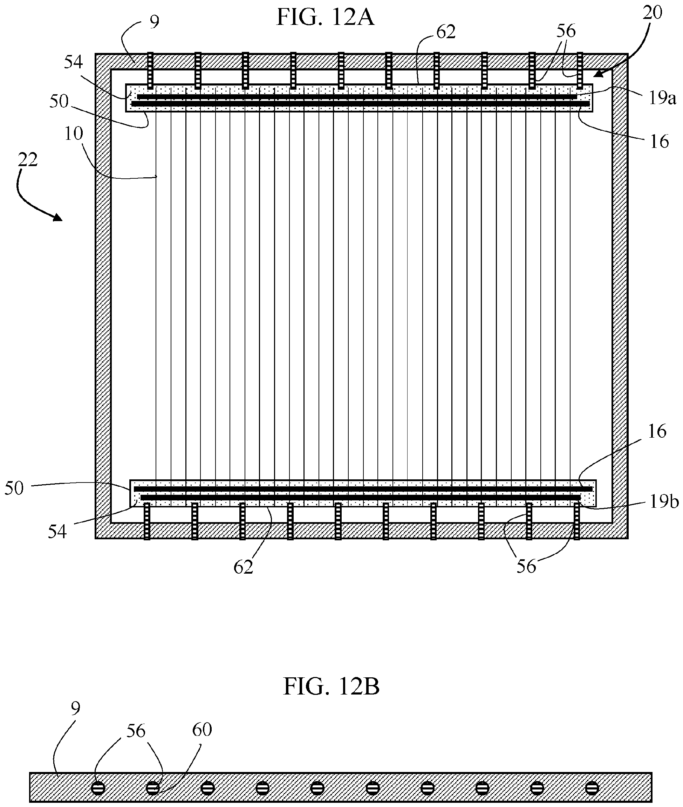

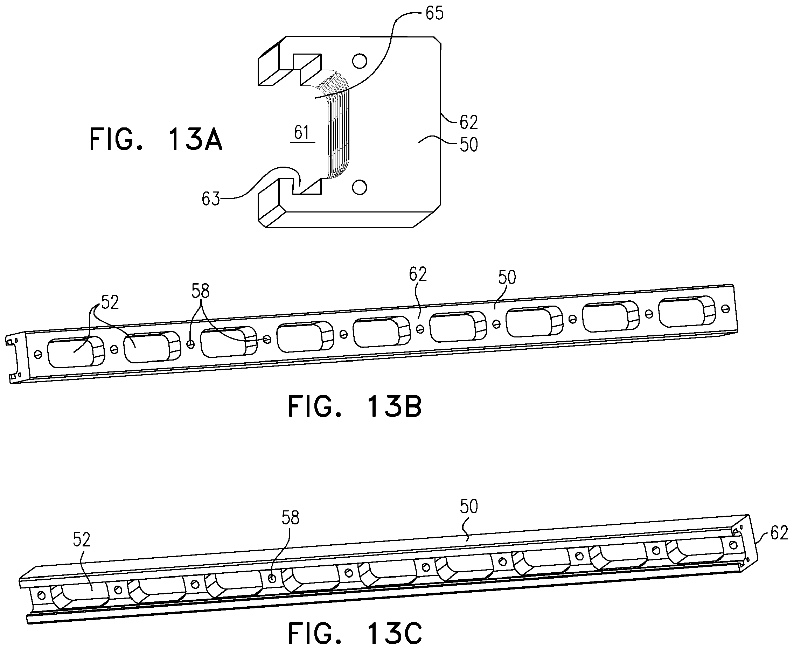

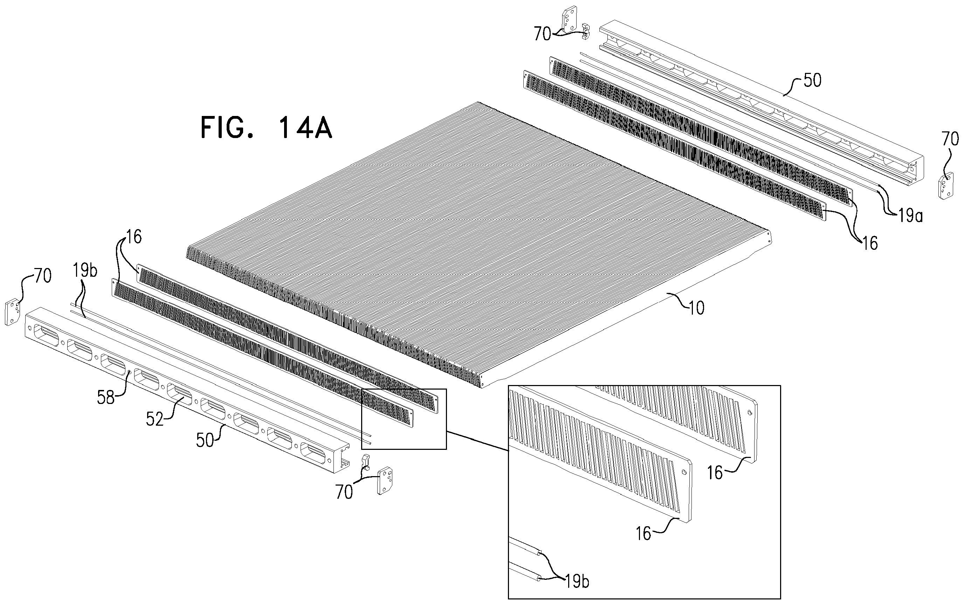

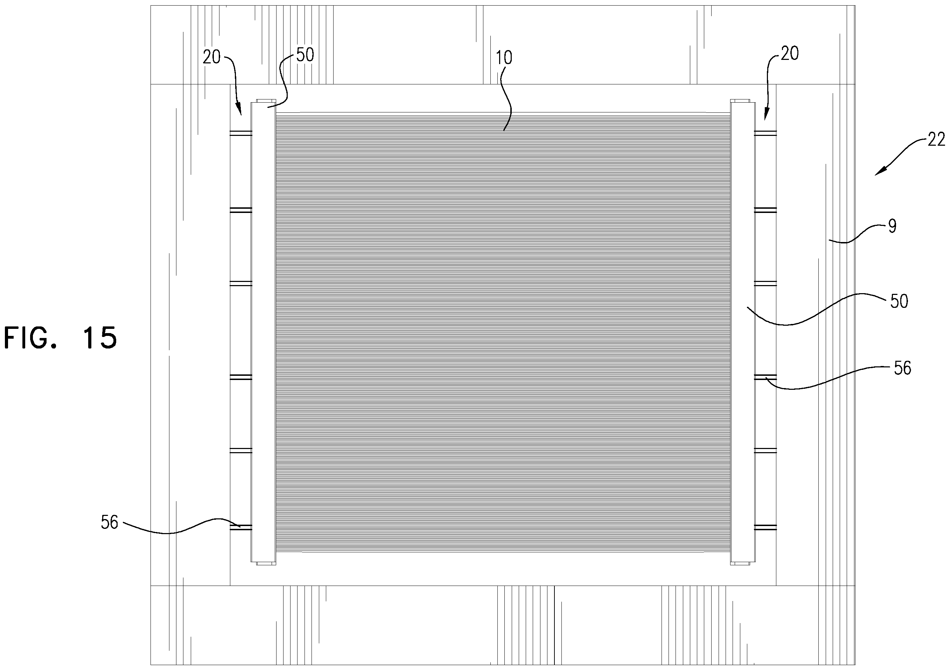

For some applications of the present invention, X-ray anti-scatter grid 22 has a configuration that is generally as described with reference to Figs. 10A-15. Figs. 10A-B, 11A-B, and 12A-B are schematic illustrations of the grid at respective stages of the manufacture of the grid, with Figs. 12A-B depicting the grid as it is typically configured once fully manufactured. Figs. 13A-C are schematic illustrations of a septa-supporting element 50 that forms a portion of pulling mechanism 20, as described in further detail hereinbelow. Figs. 14A-C are further schematic illustrations of the septa-supporting element with the ends of septa 10 and slotted plate 16 disposed within a cavity defines by the septa-supporting element. Fig. 15 is a further schematic illustration of the fully-assembled x-ray grid.

Reference is now made to Figs. 10A and 10B, which are schematic illustrations of respective views of grid 22 during an initial stage of the construction of the grid, in accordance with some applications of the present invention. Fig. 10A is a top view of the grid, and Fig. 10B is a side view of the grid taken from a side of the grid that runs along the lengths of the septa. In an initial stage of the manufacture of the grid, septa 10 are placed into slots 17 of the slotted plates 16. Typically, each end of each of the septa is placed into a respective slotted plate. As shown, the ends of the septa are inserted into slots 17 of the slotted plates such that holes 13a and 13b at the respective ends of the septa are disposed behind the slotted plates. For some applications, each of the septa defines more than one hole at each of its ends. For example, as shown in Fig. 10B, each end of the septum includes two holes.

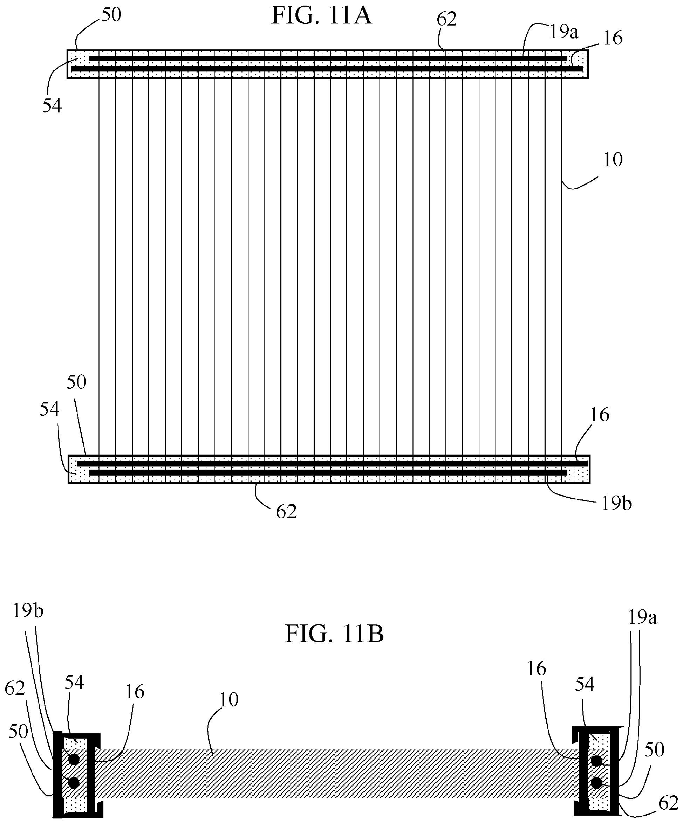

Reference is now made to Figs. 11A and 11B, which are schematic illustrations of respective views of grid 22 during a subsequent stage of the construction of the grid, in accordance with some applications of the present invention. Fig. 11A is a top view of the grid, and Fig. 11B is a side view of the grid taken from a side of the grid that runs along the length of the septa. In the subsequent stage of the manufacture of the grid, rods 19a and 19b are inserted through holes 13a and 13b of the septa. As described hereinabove, for some applications, each of the septa defines more than one hole at each of its ends. For example, as shown in Fig. 10B, each end of the septum includes two holes. For such applications, a corresponding number of rods are inserted through the holes at the ends of the septa. For example, as shown in Fig. 11B, two rods are inserted through each of the ends of each of the septa. Typically, the insertion of the rods through the holes at the ends of the septa longitudinally aligns the ends of the septa with respect to each other and/or holds the ends of the septa in place behind the slotted plates.

Subsequent to the rods being placed through the holes in the septa, septa-supporting elements 50 are placed around (e.g., are slid over) the slotted plates, the ends of the septa that lie behind the slotted plates, and the rods. Typically, a respective septa-supporting elements is placed around each of the aforementioned components at each end of the septa. Figs. 11A and 11B show the septa-supporting element disposed around the slotted plates, the ends of the septa, and the rods, at each end of the septa. Typically, a wall 62 of the septa-supporting element that faces away from the centers of the septa is shaped to define a first set of holes 52 (shown in Figs. 13B-C). Further typically, adhesive 54 is applied to the interior of the septa-supporting element via the first set of holes, such that the adhesive adheres the ends of the septa, the slotted plates, the rods and the interior of the septa-supporting element to each other.

Reference is now made to Figs. 12A and 12B, which are schematic illustrations of respective views of grid 22 once the grid has been fully constructed, in accordance with some applications of the present invention. Fig. 12A is a top view of the grid, and Fig. 12B is a side view of the grid taken from a side of the grid that runs along the ends of the septa. As shown, subsequent to the slotted plates, the ends of the septa, and the rods having been placed inside and adhered to the septa-supporting elements, the arrangement of septa and septa supporting elements are placed within frame 9, such that the frame is disposed around the perimeter of the arrangement of septa and septa supporting elements. Typically, a set of one or more (e.g., a plurality of) tension-applying elements 56 are disposed between the frame and at least one of the septa-supporting elements. For some applications, a set of one or more (e.g., a plurality of) tension-applying elements 56 are disposed between a portion of the frame and each of the septa-supporting elements. The tension-applying elements are configured to apply tension between the frame and the septa-supporting element, and to thereby apply tension to the septa. In this manner, the septa are typically maintained in taut configurations, such that, along the lengths of the septa, the longitudinal axes of the septa are maintained in a parallel configuration with respect to each other.

Typically, a wall of the septa-supporting element that faces away from the centers of the septa is shaped to define a second set of holes 58 (shown in Figs. 13B-C). Further typically, the frame defines a set of holes 60 (shown in Figs. 12B), that are configured to be aligned with the second set of holes that are defined by the septa-supporting element. For some such applications, the tension-applying elements are screws that pass through the holes 58 (defined by the septa-supporting element) and holes 60 (defined by the frame). The screws are typically tightened to a sufficient extent that the septa are maintained in taut configurations, such that, along the lengths of the septa, the longitudinal axes of the septa are maintained in a parallel configuration with respect to each other. For some applications, over the lifetime of the grid, the screws are tightened periodically in order to apply additional tension to the septa (e.g., in the event that the septa lose some tautness over time).

Reference is now made to Figs. 13A, 13B, and 13C, which are schematic illustrations of septa-supporting element 50 of pulling-mechanism 20 of x-ray grid 22, in accordance with some applications of the present invention. For some applications, the septa-supporting element is shaped to define a cavity 61 that is configured to house the slotted plates, as well as ends of the septa. As shown, the cavity typically defines a slot 63 that is shaped such as to receive the slotted plate, and a shallower portion 65 behind the slot, which is configured to receive the ends of the septa that are disposed behind the slotted plate. Thus, the slotted plate and the ends of the septa typically fit snugly within cavity 61. In accordance with the above description, the septa-supporting element typically defines a first set of holes 52 within wall 62 (which faces away from the centers of the septa). The first set of holes are configured to facilitate the application of adhesive to cavity 61, such that the adhesive adheres the ends of the septa, the slotted plates, the rods and the interior of the septa-supporting element to each other. Further typically, the septa-supporting element defines a second set of holes 58 within wall 62. The second set of holes are configured to receive the tension-applying elements 56, which are typically screws.

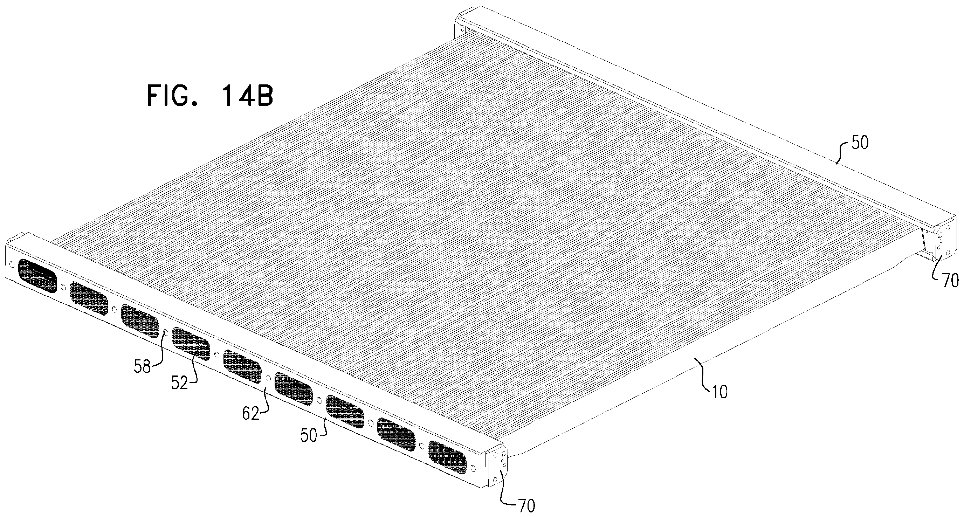

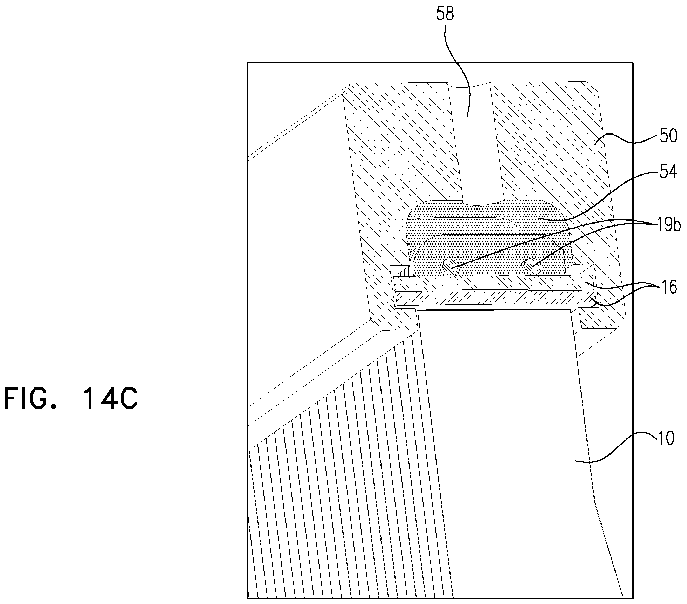

Reference is now made to Figs. 14A, 14B, and 14C, which are schematic illustrations of septa-supporting element 50 and additional portions of grid 22, in accordance with some applications of the present invention. Figs. 14A is an exploded view, which shows the septa-supporting elements 50, septa 10, slotted plates 16, rods 19a and 19b. For some applications, at each of the ends of the septa, the septa are inserted through slots 17 of a plurality of slotted plates, for example, two slotted plates (as shown in Fig. 14A), or three or more slotted plates. Typically, the slots of the slotted plates are slightly wider than the widths of the septa, in order to facilitate placement of the septa into the slots. For some such applications, the ends of the septa are inserted through slots of a plurality of slotted plates (as described), and the slotted plates are then slid with respect to each other such that the slots of the plates become misaligned with respect to each other. This typically causes the ends of the septa to become trapped within the slots of the slotted plates.

For some applications, covers 70 are configured to cover the sides of the septa-supporting element, subsequent to the ends of the septa, the slotted plates, and the rods having been placed inside cavity 61 defined by the septa-supporting elements. This is shown in Fig. 14B, which shows the septa-supporting element after the ends of the septa, the slotted plates, and the rods have been placed inside the cavity defined by the septa-supporting elements, and covers 70 have been placed on the septa-supporting elements. As described hereinabove, the slotted plate and the ends of the septa typically fit snugly within cavity 61. Furthermore, the covers are placed at the ends of cavity 61. In this manner, the ends of the septa, the slotted plates, and the rods typically become trapped inside the cavity defined by the septa-supporting elements. For some applications (not shown), at least some of the covers include elements (e.g., screws and/or in-built protrusions) that are configured to slide one of the slotted plates with respect to another, within a given septa-supporting element (e.g., as described in the above paragraph).

As described hereinabove, the ends of the septa, the slotted plates, and the rods are further typically adhered to the interior of the septa-supporting element, for example, by adhesive being applied through holes 52. Fig. 14C is a cross-sectional schematic illustration, which shows how the slotted plate and the ends of the septa are typically held within the cavity defined by the septa-supporting element.

Reference is now made to Fig. 15, which is a schematic illustration of fully-assembled X-ray anti-scatter grid 22 that includes frame 9 and pulling mechanism 20, in accordance with some applications of the present invention. As described hereinabove, for some such applications, the pulling mechanism includes tension-applying elements, which are typically screws that pass through the holes 58 (defined by the septa-supporting element) and holes 60 (defined by the frame). The screws are typically tightened to a sufficient extent that the septa are maintained in taut configurations, such that, along the lengths of the septa, the longitudinal axes of the septa are maintained in a parallel configuration with respect to each other.

It is noted that pulling mechanism 20 and frame 9 (as described with respect to Figs. 10A-15 as well as with reference to Fig. 6), typically form part of the grid during use of the grid (i.e., while the grid is being utilized with an X-ray imaging device). In other words, the pulling mechanism and the frame are typically used to apply tension to the septa over the entire lifetime of the grid, as opposed to only being used as a stage in the manufacturing process of the grid.

Typically, the pulling mechanism is used to apply more than 10,000 Newtons of tensile force (e.g., more than 15,000 Newtons of tensile force) to the septa. For some applications, the tensile force that is applied to each septum is more than 30 Newtons (e.g., more than 35 Newtons). For example, in a grid that contains approximately 400 septa, a total tensile force of 16,000 Newtons may be applied to the septa, such that approximately 40 Newtons of tensile force is applied to each septum. Typically, due to the large amount of force that is thereby applied to frame 9, frame 9 is made a strong material (e.g., aluminum), and the walls of the frame have a thickness of more than 30 mm, e.g., more than 40 mm, or more than 50 mm. For some applications, over the lifetime of the grid, the tension that is applied by the pulling mechanism is adjusted periodically in order to apply additional tension to the septa (e.g., in the event that the septa lose some tautness over time). For example, when screws are used within pulling mechanism 20 (as described hereinabove), the screws may be tightened periodically.

In the embodiment shown in Fig. 15, the septa-supporting elements 50 are disposed within the inner perimeter of frame 9, and tension-applying elements 56 (e.g., screws) extend from the septa-supporting elements to the inner perimeter of the frame. For some applications (not shown), frame 9 defines a hollow space between the walls that define the inner perimeter of the frame and the walls that define the outer perimeter of the frame. For some such applications, the septa-supporting elements are disposed within the hollow space between the walls of the frame, and the tension-applying elements 56 (e.g., screws) extend from the septa-supporting elements to the walls of the frame that define the outer perimeter of the frame. Typically, this reduces the footprint of the X-ray anti-scatter grid relative to the embodiment shown in Fig. 15.

For some applications (not shown), the upper and lower surfaces of the X-ray anti-scatter grid are covered with a thin protective layer that is configured to the protect the septa. Typically, the protective layer is made of a radiolucent polymeric material, for example, PET (Polyethylene terephthalate), such as, BoPET (Biaxially-oriented polyethylene terephthalate).

It is noted that the scope of the present invention includes combining embodiments that are described with reference to respective figures with each other. For example, the general configuration of X-ray anti-scatter grid 22 described with reference to Figs. 10A-15 may include first and second layers 11a and 11b of elongate radiopaque septa 10 (e.g., as described with reference to Figs. 3A-B). As described hereinabove, the first layer of elongate radiopaque septa includes a plurality of elongate radiopaque septa 10 arranged such that longitudinal axes of each the septa belonging to the first layer are disposed along a first direction (e.g., the x-direction), in parallel to each other. The second layer of elongate radiopaque septa includes a plurality of elongate radiopaque septa 10 arranged such that longitudinal axes of each the septa belonging to the second layer are disposed along a second direction, in parallel to each other, the second direction being generally perpendicular to the first direction (e.g., the y-direction). The first layer of radiopaque septa are disposed above the second layer of radiopaque septa, such that, when viewed along a third direction that is perpendicular to the first and second directions (e.g., the z-direction), the first and second layers of radiopaque septa define a grid. For some applications, the general configuration of X-ray anti-scatter grid 22 described with reference to Figs. 10A-15 includes only a single layer of septa, the longitudinal axes of which are parallel to each other, e.g., as shown in these figures.

Applications of the invention described herein can take the form of a computer program product accessible from a computer-usable or computer-readable medium (e.g., a non-transitory computer-readable medium) providing program code for use by or in connection with a computer or any instruction execution system, such as controller 40 and/or X-ray system processor 42. For the purpose of this description, a computer-usable or computer readable medium can be any apparatus that can comprise, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Typically, the computer-usable or computer readable medium is a non-transitory computer-usable or computer readable medium.

Examples of a computer-readable medium include a semiconductor or solid-state memory, magnetic tape, a removable computer diskette, a random-access memory (RAM), a read-only memory (ROM), a rigid magnetic disk and an optical disk. Current examples of optical disks include compact disk-read only memory (CD-ROM), compact disk-read/write (CD-R/W) and DVD.

A data processing system suitable for storing and/or executing program code will include at least one processor (e.g., controller 40 and/or X-ray system processor 42) coupled directly or indirectly to memory elements through a system bus. The memory elements can include local memory employed during actual execution of the program code, bulk storage, and cache memories which provide temporary storage of at least some program code in order to reduce the number of times code must be retrieved from bulk storage during execution.

The system can read the inventive instructions on the program storage devices and follow these instructions to execute the methodology of the applications of the invention.

Network adapters may be coupled to the processor to enable the processor to become coupled to other processors or remote printers or storage devices through intervening private or public networks. Modems, cable modem and Ethernet cards are just a few of the currently available types of network adapters.

Computer program code for carrying out operations of the present invention may be written in any combination of one or more programming languages, including an object-oriented programming language such as Java, Smalltalk, C++ or the like and conventional procedural programming languages, such as the C programming language or similar programming languages.

It will be understood that the algorithms described herein, can be implemented by computer program instructions. These computer program instructions may be provided to a processor of a general-purpose computer, special purpose computer, or other programmable data processing apparatus to produce a machine, such that the instructions, which execute via the processor of the computer (e.g., controller 40 and/or X-ray system processor 42) or other programmable data processing apparatus, create means for implementing the functions/acts specified in the algorithms described in the present application. These computer program instructions may also be stored in a computer-readable medium (e.g., a non-transitory computer-readable medium) that can direct a computer or other programmable data processing apparatus to function in a particular manner, such that the instructions stored in the computer-readable medium produce an article of manufacture including instruction means which implement the function/act specified in the algorithms. The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide processes for implementing the functions/acts specified in the algorithms described in the present application.

Controller 40 and/or X-ray system processor 42 is typically a hardware device programmed with computer program instructions to produce a special purpose computer. For example, when programmed to perform the algorithms described with reference to the figures, controller 40 and/or X-ray system processor 42 typically acts as a special purpose X-ray-grid-controller computer processor. Typically, the operations described herein that are performed by controller 40 and/or X-ray system processor 42 transform the physical state of a memory, which is a real physical article, to have a different magnetic polarity, electrical charge, or the like depending on the technology of the memory that is used. For some applications, operations that are described as being performed by a computer processor are performed by a plurality of computer processors in combination with each other.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. Apparatus for use with an X-ray system comprising:
   an X-ray anti-scatter grid (22) comprising:

   at least a first layer (11a) of elongate radiopaque septa (10) arranged such that longitudinal axes of each the septa (10) belonging to the first layer are disposed along a first direction, in parallel to each other, spaces between the septa (10) of the layer being filled with air;
   a rigid frame (9);
   two or more slotted plates (16) coupled to the rigid frame (9), each of the slotted plates defining a plurality of slots (17), each of the septa (10) passing through a respective pairs of slots defined by a pair of the slotted plates disposed on opposite sides of the frame (9) from each other, such that an orientation of each of the septa (10) with respect to the frame (9) is determined by an orientation of the corresponding pair of slots with respect to the frame (9); and
   a pulling mechanism (20) configured to apply tension to the plurality of septa (10) that are arranged in parallel to each other, during use of the X-ray anti-scatter grid, such that, along lengths of the septa (10), longitudinal axes of the septa (10) are maintained in a parallel configuration with respect to each other.

2. The apparatus according to claim 1, wherein the rigid frame (9) and the elongate septa (10) are not formed as an integral unit.

3. The apparatus according to claim 1, wherein the X-ray anti-scatter grid (22) further comprises a second layer (11b) of elongate radiopaque septa (10), arranged such that longitudinal axes of each the septa (10) belonging to the second layer (11b) are disposed along a second direction, in parallel to each other, the second direction being perpendicular to the first direction, such that, when viewed along a third direction that is perpendicular to the first and second directions, the first and second layers (11a, 11b) of radiopaque septa (10) define a grid.

4. The apparatus according to any one of the preceding claims, wherein the X-ray anti-scatter grid (22) comprises a single layer (11a) of elongate radiopaque septa (10).

5. The apparatus according to any one of the preceding claims, wherein a thickness of each of the septa (10) is between 0.04 mm and 0.1 mm.

6. The apparatus according to any one of the preceding claims, wherein a height of each of the septa (10) is between 10 mm and 20 mm.

7. The apparatus according to any one of the preceding claims, wherein, within the layer of septa (10), a distance between each of the septa (10) and adjacent septa (10) is between 0.5 mm and 1.5 mm.

8. The apparatus according to any one of the preceding claims, wherein two or more slotted plates (16) are disposed at each end of the septa (10), and wherein the slots (17) defined by the two or more slotted plates (16) disposed at each end of the septa (10) are misaligned with respect to each other, such as to trap the ends of the septa (10) within the slots (17) defined by the slotted plates (16).

9. The apparatus according to any one of the preceding claims, wherein the ends of the septa (10) defines holes (16a, 16b) therethrough, and wherein one or more rods (19a, 19b) are disposed within the holes (16a, 16b) defined by the septa (10) such as to longitudinally align the septa (10) with respect to each other.

10. The apparatus according to any one of the preceding claims, further comprising a controller (40) configured to:

    receive an input indicating a focal length of the X-ray system, and
    in response thereto, to adjust orientations of the septa (10) within the first layer.

11. The apparatus according to claim 10, wherein the controller is configured to adjust orientations of the septa (10) within the first layer such that the septa (10) are parallel to a primary X-ray beam generated by the X-ray system.

12. The apparatus according to claim 11, wherein the controller is configured to adjust orientations of the septa (10) within the first layer by adjusting orientations of the slotted plates with respect to the frame (9).

13. The apparatus according to any one of the preceding claims, wherein the pulling mechanism (20) comprises:

at least one septa-supporting element (50) that defines a cavity (61) that is configured to receive the ends of the septa (10) and one of the pair of slotted plates (16); and
a plurality of tension-applying elements (56) disposed between the septa-supporting element (50) and a portion of the frame (9).

14. The apparatus according to claim 12, wherein the plurality of tension-applying elements (56) comprises a plurality of screws, the screws being tightened to a sufficient extent that the septa (10) are maintained in taut configurations, such that, along the lengths of the septa (10), the longitudinal axes of the septa (10) are maintained in a parallel configuration with respect to each other.

15. The apparatus according to claim 12, further comprising adhesive (54) disposed within the cavity (61) defined by the septa-supporting element (50) and configured to adhere the ends of the septa (10) and the slotted plates (16) to an interior of the septa-supporting element (50).

FIG. 1A

FIG. 1B

EP 4 008 256 A1

FIG. 2

PRIOR
ART

FIG. 3A

FIG. 3B

# FIG. 4A

# FIG. 4B

EP 4 008 256 A1

FIG. 5A

16

17

z

x

FIG. 5B

16

17

y

x

FIG. 6

EP 4 008 256 A1

FIG. 7

FIG. 8

# FIG.9

EP 4 008 256 A1

FIG. 10A

FIG. 10B

FIG. 11A

FIG. 11B

## FIG. 12A

## FIG. 12B

FIG. 13A

FIG. 13B

FIG. 13C

FIG. 14A

50

70

70

19a

16

16

10

16

19b

16

58

52

50

70

19b

EP 4 008 256 A1

FIG. 14B

FIG. 14C

FIG. 15

**Europäisches Patentamt**
**European Patent Office**
**Office européen des brevets**

# EUROPEAN SEARCH REPORT

Application Number

EP 20 21 1336

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2015/318066 A1 (BECK THOMAS J [US]) 5 November 2015 (2015-11-05) * paragraphs [0046], [0057], [0055]; figures 7A,6,7 * | 1,3,5-8, 10-13,15 | INV. A61B6/00 G21K1/02 G21K1/04 |
| X | US 6 707 884 B1 (OGAWA EIJI [JP]) 16 March 2004 (2004-03-16) | 1,2,4-7, 10-12,15 | |
| Y | * figures 13A, 13B * | 1,3,5-8, 10-13,15 | |
| X | US 2011/170670 A1 (KUWABARA SHOJI [JP]) 14 July 2011 (2011-07-14) | 1,2,4-7, 9-12,15 | |
| Y | * figures 13,11 * | 8 | |
| X | JP 2008 232731 A (SHIMADZU CORP) 2 October 2008 (2008-10-02) | 1,2,4-7 | |
| Y | * paragraph [0021]; figure 1 * | 8 | |
| A | | 10-12,15 | |
| X | JP 2011 135934 A (TOSHIBA CORP; TOSHIBA MEDICAL SYS CORP; TOSHIBA MEDICAL SYS CO LTD) 14 July 2011 (2011-07-14) | 1,2,4-7, 10-12,15 | TECHNICAL FIELDS SEARCHED (IPC) |
| Y | * figure 3 * | 8 | A61B H05G G21K |
| Y | US 2010/239072 A1 (KUROCHI HARUO [JP]) 23 September 2010 (2010-09-23) | 8 | |
| A | * (11,13); figure 6 * | 10-12,15 | |
| E | US 2020/395141 A1 (LEVINSON REUVEN [IL]) 17 December 2020 (2020-12-17) * the whole document * | 1-15 | |

The present search report has been drawn up for all claims

| Place of search | Date of completion of the search | Examiner |
|---|---|---|
| Munich | 12 May 2021 | Anscombe, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 1336

This annex lists the patent family members relating to the patent documents cited in  the above-mentioned European search report.
The members are as contained in the European Patent Office EDP file on
The European Patent Office is in no way liable for these particulars which are merely given for the purpose of information.

12-05-2021

| Patent document cited in search report | | Publication date | Patent family member(s) | | Publication date |
|---|---|---|---|---|---|
| US 2015318066 | A1 | 05-11-2015 | US 2013272505 A1<br>US 2015318066 A1 | | 17-10-2013<br>05-11-2015 |
| US 6707884 | B1 | 16-03-2004 | JP 4149110 B2<br>JP 2000338254 A<br>US 6707884 B1 | | 10-09-2008<br>08-12-2000<br>16-03-2004 |
| US 2011170670 | A1 | 14-07-2011 | JP 5477000 B2<br>JP 2011145104 A<br>US 2011170670 A1 | | 23-04-2014<br>28-07-2011<br>14-07-2011 |
| JP 2008232731 | A | 02-10-2008 | NONE | | |
| JP 2011135934 | A | 14-07-2011 | JP 5683807 B2<br>JP 2011135934 A | | 11-03-2015<br>14-07-2011 |
| US 2010239072 | A1 | 23-09-2010 | JP 5383266 B2<br>JP 2010214025 A<br>US 2010239072 A1 | | 08-01-2014<br>30-09-2010<br>23-09-2010 |
| US 2020395141 | A1 | 17-12-2020 | NONE | | |

EPO FORM P0459

For more details about this annex : see Official Journal of the European Patent Office, No. 12/82

**REFERENCES CITED IN THE DESCRIPTION**

**Non-patent literature cited in the description**

- Diagnostic X-ray imaging equipment -Characteristics of general purpose and mammographic anti-scatter grids. *IEC document 60627*